# FASCICULE DE BREVET EUROPEEN

(11) **EP 3 255 427 B1**
(45) Date de publication et mention de la délivrance du brevet: **23.01.2019**
(21) Numéro de dépôt: 17171293.8
(22) Date de dépôt: 16.05.2017
(51) Int. Cl.: G01N 33/12, A22C 17/00, G01N 21/3563, G01N 21/359

(54) **INSTALLATION ET PROCÉDÉ D'AIDE À LA DÉTERMINATION DE LA QUALITÉ DE PIÈCES DE VIANDE PROVENANT D'ANIMAUX ABATTUS PRÉCÉDEMMENT**
ANLAGE UND VERFAHREN ZUR BESTIMMUNG DER QUALITÄT VON FLEISCHSTÜCKEN VON ZUVOR GESCHLACHTETEN TIEREN
FACILITY AND METHOD FOR DETERMINING THE QUALITY OF PIECES OF MEAT FROM PREVIOUSLY SLAUGHTERED ANIMALS

(30) Priorité: 07.06.2016 FR 1655212
(43) Date de publication de la demande: 13.12.2017
(73) Titulaire: Cooperl Innovation SAS, 22400 Lamballe (FR)
(72) Inventeur: LACOSTE, Anne, 35000 RENNES (FR)
(74) Mandataire: Regimbeau

(56) Documents cités:
- WO-A1-2007/000165
- FR-A1- 2 780 790
- GB-A- 2 256 924
- DATABASE WPI Week 200302 Thomson Scientific, London, GB; AN 2003-025096 XP002766238, -& JP 2002 328088 A (YUKIJIRUSHI SHOKUHIN KK) 15 novembre 2002 (2002-11-15)

## Description

### DOMAINE DE L'INVENTION

La présente invention est relative à une installation ainsi qu'à un procédé d'aide à la détermination de la qualité de pièces de viande provenant d'animaux abattus précédemment.

### ARRIERE-PLAN TECHNOLOGIQUE DE L'INVENTION

La détermination de la qualité de pièces de viande provenant d'animaux abattus précédemment et tout particulièrement des jambons de porcs répond notamment à une demande croissante des consommateurs qui recherchent toujours plus d'informations sur la qualité et la provenance des aliments qu'ils consomment.

Mais cela est aussi une préoccupation des abattoirs qui cherchent à valoriser au mieux la viande provenant d'animaux abattus.

Au-delà d'un simple tri visuel dont on perçoit très vite les limites (tâche répétitive, sélection subjective, opération chronophage), il a été proposé par le passé plusieurs techniques d'aide à la détermination de la qualité de pièces de viande provenant d'animaux abattus précédemment.

Parmi celles-ci, on note la mesure dite du "pH ultime". Toutefois, cette technique montre ses limites en particulier lorsqu'un long intervalle de temps s'écoule entre l'abattage de l'animal et la mesure de ce pH.

Une technique concurrente consiste à mesurer le spectre d'absorption dans l'infrarouge de la pièce de viande, et tout particulièrement dans le proche infrarouge (connu sous l'acronyme anglo-saxon "NIR"). En effet, la mesure d'un tel spectre rend compte du statut métabolique de la viande (c'est à dire de l'évolution métabolique de la viande en fonction du temps). Une telle technique est décrite par exemple dans FR2780790.

Cette technique s'est avérée prometteuse en ce qui concerne le "rendement technologique" des jambons, défini comme étant la capacité de la pièce de viande à retenir l'eau lors de sa cuisson. En effet, on a mis en évidence une forte corrélation entre les spectres obtenus et le rendement technologique de la viande concernée.

Et cette technique peut permettre, moyennant certaines adaptations, de déterminer un critère de qualité de viandes sur d'autres bases telles que la présence de viandes déstructurées, la présence de composés chimiques indésirables, voire de caractériser les produits en fonction de scénarios en amont (durée de réfrigération, état de stress de l'animal, type d'alimentation, etc.).

Le présent déposant a déjà mis en oeuvre cette technique. Elle requiert toutefois de nombreuses manipulations et interventions de l'opérateur, en ce qui concerne l'acquisition du spectre infrarouge et son enregistrement par des moyens informatiques.

La présente invention vise justement à rationaliser cette technique en se focalisant sur des améliorations qui permettent à l'opérateur de travailler vite tout en réduisant au maximum les manipulations qu'il a à mettre en oeuvre, ceci tout en améliorant, autant que faire se peut, la détermination de la qualité des pièces de viande.

### RESUME DE L'INVENTION

Ainsi, selon un premier aspect, la présente invention se rapporte à une installation d'aide à la détermination de la qualité de pièces de viande provenant d'animaux abattus précédemment, caractérisée par le fait qu'elle comporte :
- au moins un support d'information, ce support d'information portant, pour chaque pièce de viande, une information dite "durée post-mortem", c'est-à-dire une information caractéristique du temps écoulé depuis l'abattage de l'animal correspondant ;
- au moins un outil de lecture et d'enregistrement de cette information "durée post-mortem" ;
- au moins une sonde de lecture, reliée à un spectromètre infrarouge, ce dernier étant agencé pour mesurer le spectre d'absorption infrarouge desdites pièces de viande ;
- au moins un équipement informatique qui enregistre chaque spectre mesuré, équipement relié ou connecté à une base de données contenant un nombre prédéterminé de "spectres de référence", c'est-à-dire de spectres d'absorption infrarouge de pièces de viande associés à des durées post-mortem différentes, cette base de données contenant également parmi les spectres de référence, un certain nombre de "spectres aberrants", à savoir des spectres qui ne respectent pas des paramètres prédéfinis.
cet équipement informatique étant configuré pour :
en premier lieu et pour chaque pièce de viande testée, associer l'information "durée post-mortem" lue par ledit outil de lecture avec le spectre infrarouge mesuré associé,
en deuxième lieu, comparer ce spectre mesuré au spectre de la base de données (BDD) qui présente la même ou sensiblement la même durée post-mortem et,
en troisième lieu et seulement dans l'hypothèse où le spectre mesuré n'est pas considéré lui-même comme un "spectre aberrant", associer à la pièce de viande un critère de qualité prédéterminé en fonction de la comparaison effectuée en deuxième lieu,
   - des moyens de visualisation de l'information obtenue, en troisième lieu, par ledit équipement informatique, même si le spectre a été considéré comme aberrant.

Ainsi, grâce à cette installation, l'affichage de la valeur prédite, calculée par un algorithme de calcul réalisé à partir du spectre de mesure, permet de fournir à l'opérateur en ligne un outil d'aide à la décision pour le tri des pièces de viande.

Selon d'autres caractéristiques non limitatives et avantageuses de cette installation :
- elle comprend un algorithme configuré pour associer, à ladite pièce de viande, ledit critère de qualité.
- à chaque spectre de référence contenu dans la base de données (BDD) est associé un rendement technologique spécifique, défini comme étant la capacité de la pièce de viande à retenir l'eau lors de sa cuisson.
- lesdites pièces de viande sont des jambons de porcs.
- lesdits spectres sont des spectres d'absorption dans le proche infrarouge.
- ledit support d'information est porté par la pièce de viande ou est constitué par la pièce de viande elle-même.
- ladite information a la forme d'au moins un code-barres, d'au moins un QR-Code, d'au moins un caractère numérique, d'au moins deux caractères alphanumériques ou d'une combinaison d'au moins deux de ceux-ci.
- ledit équipement informatique est également configuré pour, en quatrième lieu, trier lesdites pièces de viande en fonction du critère de qualité qui leur a été alloué.
- ledit tri est effectué également en fonction de paramètres d'un cahier des charges.
- lesdits moyens de visualisation de l'information obtenue comprennent au moins des moyens lumineux tels que des leds.

Selon un second aspect, la présente invention se rapporte à un procédé d'aide à la détermination de la qualité de pièces de viande provenant d'animaux abattus précédemment, qui fait usage de l'installation selon l'une des revendications précédentes, caractérisé par le fait qu'il comporte au moins les étapes suivantes :
a/ lecture de ladite information "post-mortem" ;
b/ mise en contact de ladite sonde avec ladite pièce de viande et activation de celle-ci pour mesurer ledit spectre ;
c/ visualisation de l'information obtenue, en troisième lieu, par ledit équipement informatique, même si le spectre a été considéré comme aberrant ;
d/ association à la pièce de viande d'un critère de qualité prédéterminé, notamment via un algorithme.

### BREVE DESCRIPTION DES DESSINS

D'autres caractéristiques et avantages de l'invention apparaîtront à la lecture de la description suivante d'un mode de réalisation préféré de l'invention. Cette description est faite en référence aux dessins annexés dans lesquels :
- la figure 1 est une vue très schématique d'un exemple de réalisation d'une installation conforme à la présente invention ;
- la figure 2 est un exemple de réalisation d'une information post-mortem utilisable dans le cadre de présente invention :
- la figure 3 est une vue schématique de face d'une sonde de lecture reliée à un spectromètre infrarouge ;
- la figure 4 est une vue en coupe longitudinale du corps de la sonde la figure 3 ;
- la figure 5 est un organigramme permettant de comprendre le processus mis en oeuvre à l'aide de l'installation de la figure 5 ;
- les figures 6 et 7 sont deux exemples de visualisation de spectres d'absorption infrarouge lus en utilisant l'installation de l'invention, spectres comparés à des spectres aberrants préalablement enregistrés.

### DESCRIPTION DETAILLEE DE L'INVENTION

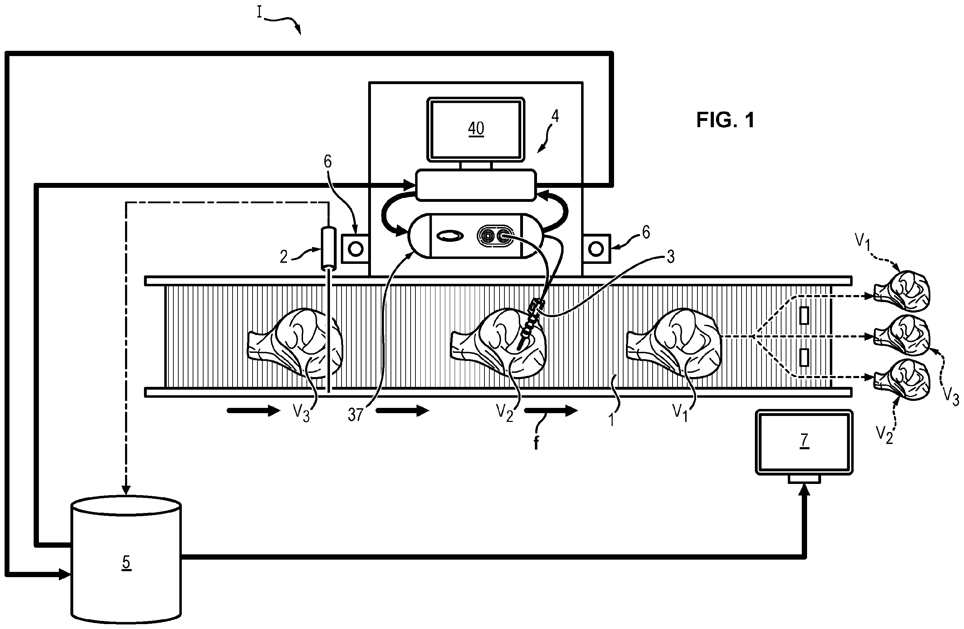

Dans l'exemple illustré à la figure 1 annexée, l'installation I d'aide à la détermination de la qualité de pièces de viande comporte un convoyeur sans fin, du type tapis roulant 1 dont le brin supérieur est configuré pour acheminer progressivement, dans le sens de la flèche F, des pièces de viande V.

Dans le mode de réalisation présenté ici, ces pièces de viande référencées V₁, V₂ et V₃ sont des jambons de porc. Mais il s'agit seulement d'un exemple dans la mesure où d'autres types de pièces de viande peuvent être également traités.

Ce convoyeur peut être celui qui est utilisé préalablement pour la découpe de ces jambons. Il peut toutefois s'agir d'un convoyeur indépendant, sur lequel les pièces de viande auront été placées spécifiquement pour être traitées dans le cadre de la présente invention.

L'installation I comporte par ailleurs un support d'information SI.

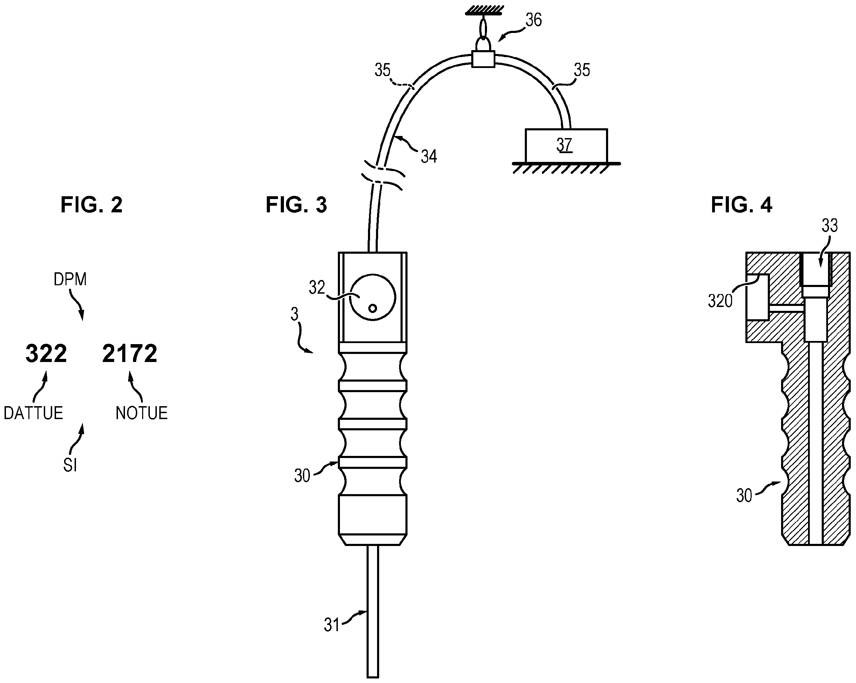

Dans le mode de réalisation de la figure 2, ce support SI est constitué par la pièce de viande elle-même et l'information qu'elle comporte est dite "durée post-mortem DPM".

Il s'agit d'une information caractéristique du temps écoulé entre l'abattage de l'animal correspondant et le prélèvement de la pièce de viande sur la carcasse de l'animal abattu. En l'occurrence, dans l'exemple présenté ici, l'information DPM comporte le quantième "322" de la date d'abattage (ou de tuerie) DATTUE. Cette information comprend également un numéro de tuerie NOTUE "2172" qui est spécifique à une session particulière d'abattage.

Dans des exemples non représentés, l'information DPM pourrait être portée par un support distinct de la pièce de viande. Il pourrait s'agir par exemple d'une étiquette collée sur la pièce de viande ou fixée à celle-ci par des moyens connus.

Toutefois, selon l'exemple présenté ici, l'information est portée de manière sûre par la pièce de viande quoi qu'il lui arrive puisque cette information est directement tatouée ou imprimée sur la peau de l'animal. Toujours dans le cadre de l'exemple présenté ici, l'information DPM est constituée exclusivement de chiffres.

Mais on pourrait envisager que cette information ait la forme d'au moins un code-barres d'au moins un QR-Code ou d'au moins deux caractères alphanumériques ou encore d'une combinaison deux de ceux-ci.

Toujours en référence à la figure 1, l'installation comporte une cellule 2 de détection de l'arrivée d'une pièce de viande, en l'occurrence V₃ dans l'exemple représenté ici. A cette fin, la cellule émet un rayonnement du genre rayonnement laser, et cette cellule constitue un outil de lecture et d'enregistrement de l'information post-mortem DPM.

Lorsque cette lecture est faite, l'enregistrement correspondant est communiqué à un équipement informatique comprenant un serveur informatique 5 qui va stocker l'information. Cette communication entre la cellule et le serveur peut se faire par liaison filaire, par Wi-Fi, ou par Internet.

A titre indicatif, le serveur utilisé est par exemple celui connu sous la référence AS/400 de la société IBM.

Par ailleurs, l'installation comporte au moins une sonde de lecture qui est plus particulièrement représentée aux figures 3 et 4 annexées. Cette sonde 3 comporte un corps 30 en plastique qui fait office de poignée de manipulation.

Il y est prévu un logement 320 de réception d'un bouton 32 d'activation de la sonde.

Le corps présente un canal longitudinal 33 qui accueille un câble de fibres optiques 35. Dans le prolongement du corps 30 s'étend une tige 31 à l'extrémité libre de laquelle affleure le câble de fibres optiques précité. Cette tige présente préférentiellement un repère qui permet à l'opérateur de visualiser la profondeur d'enfoncement de la tige 31 dans la pièce de viande.

Dans l'hypothèse où cette sonde est non pas une sonde de contact mais de pénétration, l'extrémité de la tige 31 peut être prévue en pointe.

A l'extrémité opposée, le câble 35 est renfermé dans une gaine de protection 34 qui, du côté de son extrémité distale, comprend un dispositif d'accrochage 36 qui permet, comme on le montrera plus loin, à un opérateur de pouvoir se servir de cette sonde sans avoir à la reposer entre deux manipulations.

Enfin, le câble de fibres optiques 35 est relié à un spectromètre 37 capable d'enregistrer un spectre d'absorption dans le proche infrarouge. A titre indicatif, ce spectromètre est par exemple celui commercialisé par (*à compléter s'il vous plaît*).

De plus, l'installation comporte, en plus du serveur 5, un ordinateur 4 qui fait partie intégrante de l'équipement informatique précité.

Cet équipement informatique enregistre chaque spectre mesuré avec l'installation de l'invention. Il comporte de plus ou est connecté à une base de données BDD qui contient un nombre prédéterminé de spectres de référence SR, c'est-à-dire de spectres d'absorption infrarouge de pièces de viande associés à des durées post-mortem DPM différentes.

De plus, cette base de données contient également parmi ces spectres de référence un certain nombre de spectres aberrants SA. On entend par l'expression "spectre aberrant" un spectre qui ne respecte pas des paramètres prédéfinis. A titre d'exemple, ces paramètres peuvent consister en une anomalie sur le temps de contact entre la sonde et la pièce de viande, le positionnement de la sonde (si la sonde n'est pas mise en contact avec le muscle), le spectre correspondant est alors aberrant), etc.

L'installation comporte par ailleurs des moyens de visualisation de l'information qui sera délivrée au final par l'installation, information sur le caractère acceptable ou non de la pièce de viande testée.

Ces moyens de visualisation 6 sont par exemple constitués par des moyens lumineux, tels que des leds qui, selon l'information à délivrer, afficheront une lumière de couleurs différentes, par exemple orange, rouge ou verte.

Plus globalement, le procédé de la détermination de la qualité de pièces de viande conforme à l'invention comprend au moins les étapes suivantes qui consistent à :
a) lire l'information de durée post-mortem DPM ;
b) mettre en contact de la sonde 3 avec la pièce de viande V, et à activer de celle-ci pour mesurer le spectre d'absorption infrarouge ;
c) la visualisation de l'information obtenue, même si le spectre a été considéré comme aberrant ;
d) association à la pièce de viande d'un critère de qualité prédéterminé, notamment via un algorithme.

En tout état de cause, le principe à la base de l'invention est, pour chaque pièce de viande, d'acquérir le spectre d'information infrarouge par le spectromètre 37, de transférer ce spectre à un équipement informatique qui va en parallèle collecter l'information de durée post-mortem, en vue d'afficher un résultat relatif à la qualité de la pièce de viande testée.

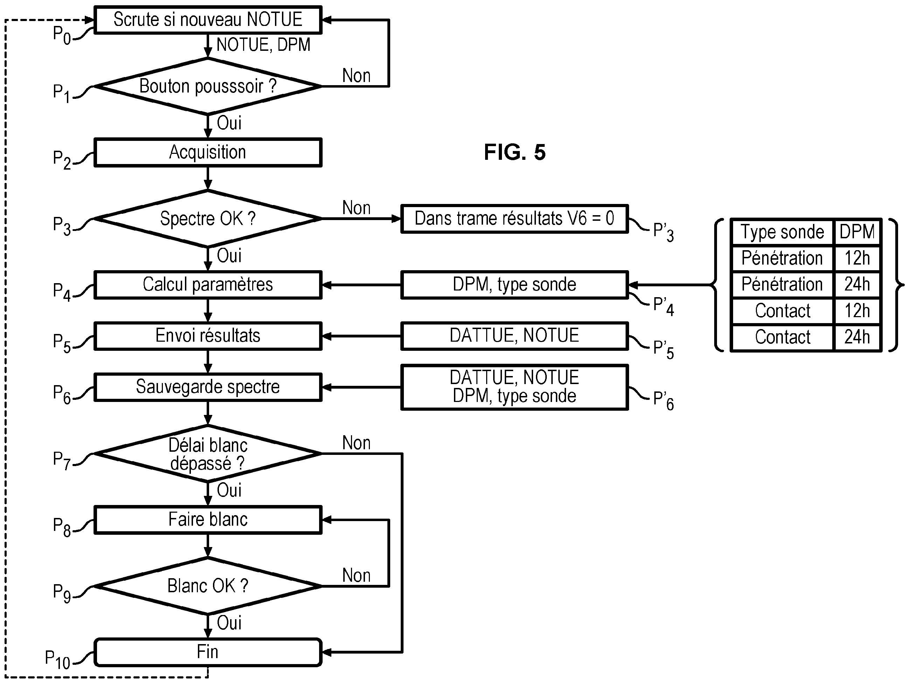

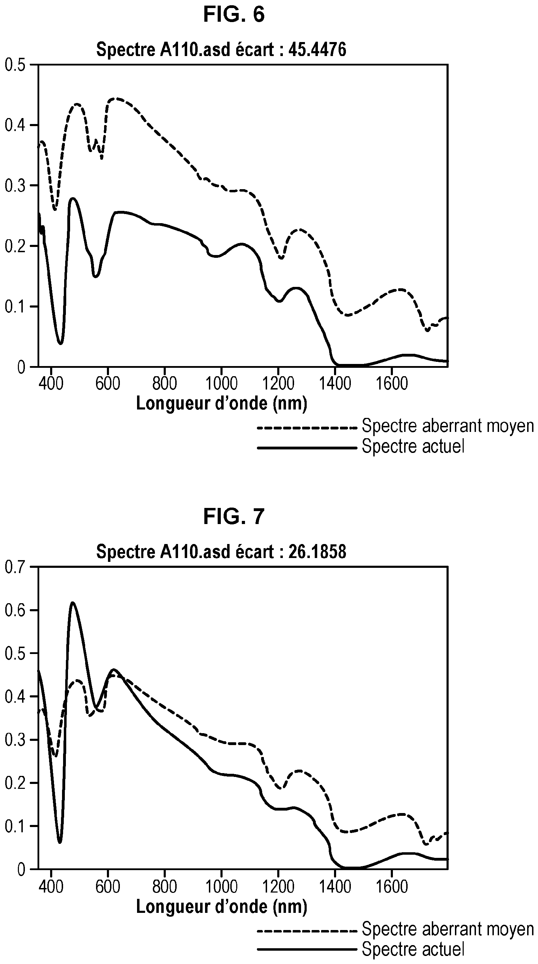

En se reportant à la figure 5, et en parallèle avec la figure 6, on peut illustrer ci-après le déroulement de ce processus.

Ainsi, dans une étape P₀, la cellule 2 est en attente de la détection d'une nouvelle pièce de viande V₃ en vue de détecter l'information DPM.

Le système attend alors que le bouton d'actionnement 32 de la sonde 3 soit activé. Ceci correspond au poste P₁ de la figure 5. Dans l'hypothèse où celui-ci est effectivement activé, on passe au poste P₂ d'acquisition du spectre via la sonde qui travaille en collaboration avec le spectromètre 37.

Lorsque le spectre est enregistré (poste P₃), l'équipement informatique le compare à ceux présents dans la base de données BDD et vérifie si ce spectre est similaire à un spectre aberrant SA.

Pour ce faire, l'équipement informatique est par exemple configuré pour calculer l'aire sous le spectre, enregistrer sa forme et comparer ces données à celles de la "collection" de spectres aberrants.

Dans cette éventualité, une information relative à la mesure d'un spectre aberrant est affichée sur l'interface visuelle 40 de l'ordinateur 4.

Dans l'hypothèse où ce spectre est correct, le système informatique récupère l'information DPM déjà obtenue ainsi que le type de sonde (de contact ou de pénétration).

Au poste P₅, les résultats sont affichés sur l'interface visuelle 40 et, au poste P'₅, l'information DPM est alors enregistrée. Au poste P₆, il en est de même pour le spectre qui a été mesuré.

En tout état de cause, si l'état du spectre est bon, les paramètres ou valeurs de prédiction de la qualité de la viande sont calculés grâce à des équations (algorithmes) de prédiction développées à cet effet.

De plus, dès lors que d'autres critères de sélection auront été chargés dans le système informatique (tenant par exemple compte d'un Cahier des Charges), un tri encore plus sélectif parmi les pièces de viande pourra être mis en oeuvre.

Dans une étape ultérieure, au poste P₇, le système détecte si un temps correct est utilisé pour une nouvelle détection de pièce de viande. Si ce délai est dépassé, on procède alors à un "blanc", c'est-à-dire une reconfiguration de la sonde. Dans le cas contraire, on arrive directement à l'étape P₁₀, c'est-à-dire à la fin du processus, et on réitère alors ce processus en mettant en oeuvre le poste P₀.

La reconfiguration de la sonde 3 (ou étalonnage) peut être réalisée en prenant comme matériau de référence celui connu sous la marque "Spectralon".

En se reportant aux figures 6 et 7, on note, sur la gauche et en partie inférieure du schéma, le profil d'un spectre lu à l'aide de l'installation de l'invention et, en partie supérieure, réalisée en traits interrompus, le profil d'un spectre aberrant déjà enregistré dans la base de données BDD. L'équipement informatique est en mesure de détecter que le spectre lu n'empiète pas sur le spectre aberrant, de sorte que ce spectre lu est considéré comme correct. Par contre, dans le cas de la figure 7, on constate un chevauchement entre le spectre lu et le spectre aberrant affiché, de sorte qu'il est délivré une information relative au spectre lu considéré dans ce cas spécifique comme un spectre aberrant.

A titre indicatif, il se passe environ quatre secondes entre le positionnement de la sonde 3 et l'affichage des résultats à l'aide de l'équipement informatique. Ainsi, le positionnement de la sonde sur la pièce de viande prendre environ une seconde. Il faut sensiblement le même temps pour acquérir le spectre grâce au spectromètre 37.

Le traitement par l'équipement informatique dure moins d'une seconde et le reste du temps est consacré à l'envoi des résultats. Toutefois, à compter du traitement par l'équipement informatique, l'opérateur peut retirer la sonde et procéder à son nettoyage.

On peut ainsi espérer un traitement de 900 pièces de viande à l'heure.

Grâce à l'invention, on peut procéder au tri en ligne de la matière première en fonction de l'historique du processus de fabrication de la pièce découpée qui prend en compte le statut métabolique de la viande.

Par ailleurs, on peut reconnaître la présence d'une mesure aberrante. De plus, l'opérateur voit la préhension de la pièce de viande facilitée et améliore son geste lors des prises de mesure. Cette mesure est particulièrement fiable, notamment par la temporisation et le repère sur la profondeur de pénétration de la sonde 3. Enfin, cette dernière est particulièrement facile à nettoyer et robuste du fait des moyens de protection qu'elle comporte.

## Revendications

1. Installation (I) d'aide à la détermination de la qualité de pièces de viande provenant d'animaux abattus précédemment, **caractérisée par le fait qu'**elle comporte :
- au moins un support d'information (SI), ce support d'information portant, pour chaque pièce de viande (V ; V₁ ; V₂ ; V₃), une information dite "durée post-mortem" (DPM), c'est-à-dire une information caractéristique du temps écoulé depuis l'abattage de l'animal correspondant ;
- au moins un outil (2) de lecture et d'enregistrement de cette information "durée post-mortem" (DPM) ;
- au moins une sonde (3) de lecture, reliée à un spectromètre infrarouge (37), ce dernier étant agencé pour mesurer le spectre (S) d'absorption infrarouge desdites pièces de viande (V ; V₁ ; V₂ ; V₃) ;
- au moins un équipement informatique (4, 5) qui enregistre chaque spectre mesuré (SM), équipement relié ou connecté à une base de données (BDD) contenant un nombre prédéterminé de "spectres de référence" (SR), c'est-à-dire de spectres d'absorption infrarouge de pièces de viande associés à des durées post-mortem différentes, cette base de données contenant également parmi les spectres de référence, un certain nombre de "spectres aberrants" (SA), à savoir des spectres qui ne respectent pas des paramètres prédéfinis.
cet équipement informatique (4, 5) étant configuré pour :
en premier lieu et pour chaque pièce de viande testée (V ; V₁ ; V₂ ; V₃), associer l'information "durée post-mortem" (DPM) lue par ledit outil de lecture (2) avec le spectre infrarouge mesuré associé (SM),
en deuxième lieu, comparer ce spectre mesuré (SM) au spectre (SR) de la base de données (BDD) qui présente la même ou sensiblement la même durée post-mortem et,
en troisième lieu et seulement dans l'hypothèse où le spectre mesuré (SM) n'est pas considéré lui-même comme un "spectre aberrant" (SA), associer à la pièce de viande (V ; V₁ ; V₂ ; V₃) un critère de qualité prédéterminé en fonction de la comparaison effectuée en deuxième lieu,
- des moyens de visualisation (6) de l'information obtenue, en troisième lieu, par ledit équipement informatique (4, 5), même si le spectre (S) a été considéré comme aberrant.

2. Installation selon la revendication 1, **caractérisée par le fait qu'**elle comprend un algorithme configuré pour associer, à ladite pièce de viande, ledit critère de qualité.

3. Installation selon l'une des revendications précédentes, **caractérisée par le fait qu'**à chaque spectre de référence contenu dans la base de données (BDD) est associé un rendement technologique spécifique, défini comme étant la capacité de la pièce de viande à retenir l'eau lors de sa cuisson.

4. Installation (I) selon l'une des revendications 1 à 3, **caractérisée par le fait que** lesdites pièces de viande (V ; V₁ ; V₂ ; V₃) sont des jambons de porcs.

5. Installation (I) selon l'une des revendications précédentes, **caractérisée par le fait que** lesdits spectres sont des spectres d'absorption dans le proche infrarouge.

6. Installation (I) selon l'une des revendications précédentes, **caractérisée par le fait que** ledit support d'information (SI) est porté par la pièce de viande (V ; V₁ ; V₂ ; V₃) ou est constitué par la pièce de viande (V ; V₁ ; V₂ ; V₃) elle-même.

7. Installation selon l'une des revendications précédentes, **caractérisée par le fait que** ladite information (DPM) a la forme d'au moins un code-barres, d'au moins un QR-Code, d'au moins un caractère numérique, d'au moins deux caractères alphanumériques ou d'une combinaison d'au moins deux de ceux-ci.

8. Installation selon l'une des revendications précédentes, **caractérisée par le fait que** ledit équipement informatique (4, 5) est également configuré pour, en quatrième lieu, trier lesdites pièces de viande (V ; V₁ ; V₂ ; V₃) en fonction du critère de qualité qui leur a été alloué.

9. Installation selon la revendication 8, **caractérisé par le fait que** ledit tri est effectué également en fonction de paramètres d'un cahier des charges.

10. Installation selon l'une des revendications précédentes, **caractérisée par le fait que** lesdits moyens de visualisation (6) de l'information obtenue comprennent au moins des moyens lumineux tels que des leds.

11. Procédé d'aide à la détermination de la qualité de pièces de viande (V ; V₁ ; V₂ ; V₃) provenant d'animaux abattus précédemment, qui fait usage de l'installation (I) selon l'une des revendications précédentes, **caractérisé par le fait qu'**il comporte au moins les étapes suivantes :
a/ lecture de ladite information "post-mortem" (DPM) ;
b/ mise en contact de ladite sonde (3) avec ladite pièce de viande (V ; V₁ ; V₂ ; V₃) et activation de celle-ci pour mesurer ledit spectre ;
c/ visualisation de l'information obtenue, en troisième lieu, par ledit équipement informatique, même si le spectre (S) a été considéré comme aberrant (SA) ;
d/ association à la pièce de viande (V ; V₁ ; V₂ ; V₃) d'un critère de qualité prédéterminé, notamment via un algorithme.

## Patentansprüche

1. Anlage (I) zur Hilfe bei der Bestimmung der Qualität von Fleischstücken von zuvor geschlachteten Tieren, **dadurch gekennzeichnet, dass** sie Folgendes umfasst:
- zumindest einen Informationsträger (SI), wobei dieser Informationsträger für jedes Fleischstück (V; V₁; V₂; V₃) eine Information enthält, die als "Post-mortem-Zeit" (DPM) bezeichnet wird, das heißt eine Information, die charakteristisch für die seit der Schlachtung des entsprechenden Tieres vergangene Zeit ist;
- zumindest ein Werkzeug (2) zum Lesen und Aufzeichnen dieser "Post-mortem-Zeit" (DPM) Information;
- zumindest eine Lesesonde (3), die mit einem Infrarotspektrometer (37) verbunden ist, wobei Letzteres zum Messen des Infrarot-Absorptionsspektrums (S) der Fleischstücke (V; V₁; V₂; V₃) gestaltet ist;
- zumindest eine IT-Ausrüstung (4, 5), die jedes gemessene Spektrum (SM) aufzeichnet, wobei die Ausrüstung mit einer Datenbank (BDD) verbunden oder daran angeschlossen ist, die eine vorbestimmte Anzahl von "Referenzspektren" (SR) enthält, das heißt Infrarot-Absorptionsspektren von Fleischstücken, denen verschiedene Post-mortem-Zeiten zugeordnet sind, wobei diese Datenbank auch unter den Referenzspektren eine gewisse Anzahl von "abwegigen Spektren" (SA) enthält, das heißt Spektren, die vorgegebene Parameter nicht befolgen,
wobei diese IT-Ausrüstung (4, 5) wie folgt konfiguriert ist:
erstens und für jedes geprüfte Fleischstück (V; V₁; V₂; V₃) die vom Lesewerkzeug (2) gelesene Information zur "Post-mortem-Zeit" (DPM) dem zugehörigen gemessenen Infrarotspektrum (SM) zuordnen,
zweitens dieses gemessene Spektrum (SM) mit dem Spektrum (SR) der Datenbank (BDD) vergleichen, das die gleiche oder im Wesentlichen die gleiche Post-Mortem-Zeit aufweist und,
drittens, und nur, wenn das gemessene Spektrum (SM) nicht selbst als "abwegiges Spektrum" (SA) betrachtet wird, dem Fleischstück (V; V₁; V₂; V₃) ein je nach dem unter zweitens angestellten Vergleich vorbestimmtes Qualitätskriterium zuordnen,
- Anzeigemittel (6) für die unter drittens von der IT-Ausrüstung (4, 5) erhaltene Information, selbst wenn das Spektrum (S) als abwegig betrachtet worden ist.

2. Anlage nach Anspruch 1, **dadurch gekennzeichnet, dass** sie einen Algorithmus umfasst, der so konfiguriert ist, dass er dem Fleischstück das Qualitätskriterium zuordnet.

3. Anlage nach einem beliebigen der vorstehenden Ansprüche, **dadurch gekennzeichnet, dass** jedes in der Datenbank (BDD) enthaltene Referenzspektrum mit einem spezifischen technologischen Ertrag verbunden ist, der als die Fähigkeit des Fleischstücks definiert ist, beim Kochvorgang Wasser zu binden.

4. Anlage (I) nach einem beliebigen der vorstehenden Ansprüche 1 bis 3, **dadurch gekennzeichnet, dass** die Fleischstücke (V; V₁; V₂; V₃) Schweineschinken sind.

5. Anlage (I) nach einem beliebigen der vorstehenden Ansprüche, **dadurch gekennzeichnet, dass** die Spektren Absorptionsspektren im nahen Infrarot sind.

6. Anlage (I) nach einem beliebigen der vorstehenden Ansprüche, **dadurch gekennzeichnet, dass** der Informationsträger (SI) vom Fleischstück (V; V₁; V₂; V₃) getragen wird oder aus dem Fleischstück (V; V₁; V₂; V₃) selbst besteht.

7. Anlage nach einem beliebigen der vorstehenden Ansprüche, **dadurch gekennzeichnet, dass** die Information (DPM) die Form von zumindest einem Barcode, zumindest einem QR-Code, zumindest einem numerischen Zeichen, zumindest zwei alphanumerischen Zeichen oder einer Kombination von zumindest zwei von ihnen hat.

8. Anlage nach einem beliebigen der vorstehenden Ansprüche, **dadurch gekennzeichnet, dass** die IT-Ausrüstung (4, 5) auch so konfiguriert ist, dass sie viertens die Fleischstücke (V; V₁; V₂; V₃) je nach dem ihnen zugeordneten Qualitätskriterium sortiert.

9. Anlage nach Anspruch 8, **dadurch gekennzeichnet, dass** die Sortierung auch nach den Parametern eines Lastenheftes durchgeführt wird.

10. Anlage nach einem beliebigen der vorstehenden Ansprüche, **dadurch gekennzeichnet, dass** die Anzeigemittel (6) für die erhaltenen Informationen zumindest Lichtmittel wie LEDs umfassen.

11. Verfahren zur Hilfe bei der Bestimmung der Qualität von Fleischstücken (V; V₁; V₂; V₃) von zuvor geschlachteten Tieren, bei dem die Anlage (I) nach einem beliebigen der vorstehenden Ansprüche benutzt wird, **dadurch gekennzeichnet, dass** es zumindest die folgenden Schritte umfasst:
a/ Lesen der "Post-mortem-Information" (DPM);
b/ Verbinden der Sonde (3) mit dem Fleischstück (V; V₁; V₂; V₃) und Aktivieren derselben zum Messen des Spektrums;
c/ Anzeigen der unter drittens von der Ausrüstung erhaltenen Information, selbst wenn das Spektrum (S) als abwegig (SA) betrachtet worden ist;
d/ Zuordnen eines insbesondere über einen Algorithmus vorbestimmten Qualitätskriteriums zu einem Fleischstück (V; V₁; V₂; V₃).

## Claims

1. An installation (I) for helping to determine the quality of pieces of meat originating from previously slaughtered animals, **characterized by** the fact that it includes:
- at least one information medium (SI), this information medium carrying, for each piece of meat (V; V₁; V₂; V₃), an information called "post-mortem time" (DPM) information, i.e. an information characteristic of the time elapsed since the slaughter of the corresponding animal;
- at least one tool (2) for reading and recording this "post-mortem time" (DPM) information;
- at least one reading probe (3), linked to an infrared spectrometer (37), the latter being arranged to measure the infrared absorption spectrum (S) of said pieces of meat (V; V₁; V₂; V₃);
- at least one computer equipment (4, 5) which records each measured spectrum (SM), which equipment is linked or connected to a database (BDD) containing a predetermined number of "reference spectra" (SR), i.e. infrared absorption spectra of associated pieces of meat at different post-mortem times, this database containing also among the reference spectra, a certain number of "aberrant spectra" (SA), namely spectra which do not respect predefined parameters,
this computer equipment (4, 5) being configured to:
firstly, and for each tested piece of meat (V; V₁; V₂; V₃), associate the "post-mortem time" (DPM) information read by said reading tool (2) with the associated measured infrared spectrum (SM),
secondly, compare this measured spectrum (SM) with the spectrum (SR) of the database (BDD) that presents the same or substantially the same post-mortem time and,
thirdly and only in the event that the measured spectrum (SM) is not considered itself as being an "aberrant spectrum" (SA), associate with the piece of meat (V; V₁; V₂; V₃) a predetermined quality criterion according to the comparison performed secondly,
- means for displaying (6) the information obtained, thirdly, by said computer equipment (4, 5), even if the spectrum (S) has been considered as aberrant.

2. The installation according to claim 1, **characterized by** the fact that it comprises an algorithm configured to associate, with said piece of meat, said quality criterion.

3. The installation according to any of the preceding claims, **characterized by** the fact that, with each reference spectrum contained in the data base (BDD) is associated a specific technological performance, defined as the ability of the piece of meat to retain water during its cooking.

4. The installation (I) according to any of claims 1 to 3, **characterized by** the fact that said pieces of meat (V; V₁; V₂; V₃) are ham.

5. The installation (I) according to any of the preceding claims, **characterized by** the fact that said spectra are absorption spectra in the near infrared.

6. The installation (I) according to any of the preceding claims, **characterized by** the fact that said information medium (SI) is carried by the piece of meat (V; V₁; V₂; V₃) or is composed of the piece of meat (V; V₁; V₂; V₃) itself.

7. The installation according to any of the preceding claims, **characterized by** the fact that said information (DPM) has the form of at least one bar code, at least one QR-Code, at least one numeric character, at least two alphanumeric characters or a combination of at least two of these.

8. The installation according to any of the preceding claims, **characterized by** the fact that said computer equipment (4, 5) is also configured, fourthly, to sort said pieces of meat (V; V₁; V₂; V₃) according to the quality criterion that has been allocated thereto.

9. The installation according to claim 8, **characterized by** the fact that said sorting is also performed according to parameters of specifications.

10. The installation according to any of the preceding claims, **characterized by** the fact that said means for displaying (6) the obtained information comprise at least light means such as LEDs.

11. A method for helping to determine the quality of pieces of meat (V; V₁; V₂; V₃) originating from previously slaughtered animals, which makes use of the installation (I) according to any of the preceding claims, **characterized by** the fact that it includes at least the following steps:
a/ reading said "post-mortem time" (DPM) information;
b/ putting said probe (3) into contact with said piece of meat (V; V₁; V₂; V₃) and activating it to measure said spectrum;
c/ displaying the obtained information, thirdly, by said computer equipment, even if the spectrum (S) has been considered as aberrant (SA);
d/ associating with the piece of meat (V; V₁; V₂; V₃) a predetermined quality criterion, in particular via an algorithm.
